(19) Europäisches Patentamt
European Patent Office
Office européen des brevets

(11) Publication number: **0 312 813 A2**

# (12) EUROPEAN PATENT APPLICATION

(21) Application number: 88115979.2

(22) Date of filing: 28.10.85

(51) Int. Cl.4: C07D 498/04 , //C12P17/18, (C07D498/04,263:00,205:00)

(30) Priority: 27.10.84 ES 537157
27.10.84 ES 537158
27.10.84 ES 537159
27.10.84 ES 537160

(43) Date of publication of application: 26.04.89 Bulletin 89/17

(60) Publication number of the earlier application in accordance with Art.76 EPC: 0 182 522

(84) Designated Contracting States: AT BE CH DE FR GB IT LI LU NL SE

(71) Applicant: Antibioticos S.A.
Bravo Murillo 38
28015 Madrid(ES)

(72) Inventor: Puentes, Jose Luis Fernandez c/o Antibioticos S.A.
Bravo Murillo, 38
E-28015 Madrid(ES)
Inventor: Valle, Miguel Angel Moreno c/o Antibioticos S.A.
Bravo Murillo, 38
E-28015 Madrid(ES)
Inventor: Maldonado, Francisco SaltoSc/o Antibioticos S.A.
Bravo Murillo, 38
E-28015 Madrid(ES)
Inventor: Izard, Tomas Olleros c/o Antibioticos S.A.
Bravo Murillo, 38
E-28015 Madrid(ES)
Inventor: Pla, Luis Costa c/o Antibioticos S.A.
Bravo Murillo, 38
E-28015 Madrid(ES)
Inventor: Sousa-Faro, Jose Maria F. c/o Antibioticos S.A.
Bravo Murillo, 38
E-28015 Madrid(ES)

(74) Representative: Ablewhite, Alan James et al
MARKS & CLERK 57/60 Lincoln's Inn Fields
London WC2A 3LS(GB)

## (54) Preparation of clavulanic acid and its salts and esters.

(57) The present invention relates to a process for obtaining clavulanic acid from solution by mixing dissolved lithium 2-ethylhexanoate with the solution, isolating the lithium clavulanate obtained and optionally converting the product to another salt or ester.

EP 0 312 813 A2

# PREPARATION OF CLAVULANIC ACID AND ITS SALTS AND ESTERS

The present invention relates to the preparation of clavulanic acid, and pharmaceutically acceptable salts and esters thereof.

Clavulanic acid, (2R,5R,Z)-3-(2-hydroxyethylidene)-7-oxo-4-oxa-1-azabicyclo-[3,2,0]-heptane-2-carboxylic acid, is a known compound of structure:

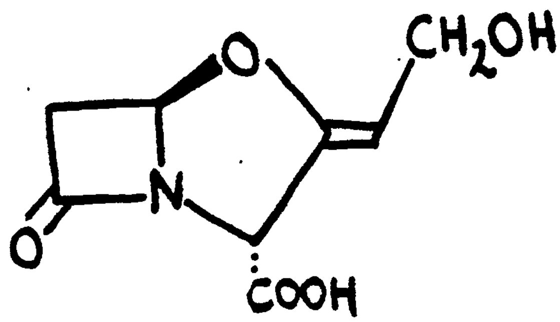

This compound, and its salts and esters, act as beta-lactamase inhibitors, and inhibit the action of beta-lactamases produced by both gram-positive and gram-negative organisms. They are therefore employed in pharmaceutical compositions to prevent inactivation of beta-lactam antibiotics. Furthermore, clavulanic acid is itself believed to have an anti-bacterial activity.

Clavulanic acid is produced from various strains of micro-organism, for example the strains belonging to the genus Streptomyces such as S.clavuligerus NRRL 3585, S. jumoninensis NRRL 5741. S. katsurahamanus IFO 13716, and Streptomyces sp. P6621 FERM 2804.

Modifications in the fermentation process have been used to increase the yield of clavulanic acid. For example, it is now known that strict pH control to a range of 6.3 to 6.7 can markedly increase the yield.

More frequently, attempts to increase yield have involved the purification process. For example, clavulanic acid salts are generally more stable than the free acid. Consequently, salts such as alkali metal and alkaline-earth metal salts have been employed to enhance the extraction yield.

One possible extraction technique is based upon the differential solubility between aqueous and organic phases of clavulanic acid and its salts. Extraction is followed by several purifications in adsorbent material, and is thus lengthy and expensive.

The direct crystallization of clavulanic acid from the organic phase is therefore desirable. In one such method, clavulanic acid is directly crystallized from the organic phase by addition of an amine such as t-butylamine, followed by conversion to a potassium salt by exchange with a base.

The lithium salt is also considered a useful intermediate in the preparation of clavulanic acid, due to its low solubility and its easy conversion to salts or esters, acceptable for pharmaceutical formulations. Reaction of a solution of clavulanic acid with an aqueous ionic lithium compound, yields lithium clavulanate. Another route to lithium clavulanate utilizes the adsorption of filtered broth in granulated carbon, an ion-exchange resin treatment and crystallization of lithium clavulanate.

Crystallization from isopropanol of lithium clavulanate, after direct adsorption of the fermentation broth in the resin, has also been suggested. Such a process is difficult and gives poor yields.

Hence, the fermentation and purification methods of the available technology have produced impure clavulanic acid in yields which are relatively low.

The present invention provides a process for purifying clavulanic acid from solution as its lithium salt, and for optionally converting the lithium clavulanate to other salts or to esters of clavulanic acid.

Thus, according to a first aspect of the present invention there is provided a method for the production of clavulanic acid wherein the clavulanic acid is purified by:

(i) mixing a fermentation broth, containing the dissolved impure clavulanic acid, with dissolved lithium 2-ethylhexanoate, the acid and the salt each being preferably dissolved in a water-immiscible solvent, more preferably the same solvent;

(ii) isolating lithium clavulanate; and

(iii) optionally converting the lithium salt to another salt or to an ester.

By such a process it is possible to obtain both enhanced yield and purity. It will be appreciated that the process is of general applicability to the purification of clavulanic acid fermentation broths, such fermentations readily leading to solutions of impure clavulanic acid in a water-immiscible solvent, suited for use in the step (i).

The prefered organic solvents are ethyl acetate, methyl acetate, amyl acetate, methyl isobutyl ketone or n-butyl alcohol, with n-butyl alcohol being most preferable.

In working up the broth, the solids are preferably first removed from the fermentation broth by filtration or centrifugation. It is also preferred that the broth is acidified to a pH of 1 to 3, preferably around pH 2, and clavulanic acid extracted by adding a water-immiscible solvent, with the two phases being separated for example by centrifugation, thereby giving the clavulanic acid in the water-immiscible phase.

Extraction of the broth is preferably conducted at a temperature in the range 4 to 10 °C, with the purpose of minimizing losses due to decomposition of the clavulanic acid.

Following extraction, crystallisation may be carried out by the addition of a solution of lithium 2-ethylhexanoate, suitably prepared in known manner. For preference, the concentration of the clavulanic acid in the organic solvent is more than 10 mg/ml. Crystallization is best performed at low temperatures, normally between 4 and 10 °C. Since lithium clavulanate crystallizes with one quarter of a molecule of water, it is recommended that crystallization is performed in the presence of a small concentration of water, such as 2 to 4% in the organic solvent. Excess lithium 2-ethylhexanoate is preferably used. Addition can take place slowly under stirring and cooling. The stirring is best maintained for a period of time after all lithium 2-ethylhexanoate has been added.

The resultant lithium clavulanate can then be further processed, and is preferably filtered through a glass plate, washed with isopropanol, acetone and ether, and dried under vacuum.

The lithium clavulanate may be recrystallized. The recrystallization can be carried out by adding ato aqueous lithium clavulanate an excess of an organic solvent in which the solubility of the lithium salt is very low, preferably isopropanol. Alternatively, and more preferably, a concentrated solution of another lithium salt is added to aqueous lithium clavulanate to cause the recrystallization. Suitable lithium salts include organic or inorganic lithium salts having high solubility in water. Lithium chloride is most preferable. Suitably, the concentration of the lithium salt solution is more than 50%, and sufficient solution is added so that the final concentration is more than 25%. Addition of the lithium salt solution preferably takes place slowly, while the lithium clavulanate solution is stirred.

The recrystallized lithium clavulanate may be further purified, for instance it can be separated by filtration, washed with acetone and dried under vacuum, thereby obtaining a yield of more than 80% and a purity above 85%.

Lithium clavulanate prepared in accordance with thls invention can be converted to other salts by ion-exchange procedures, using cationic exchange resins, in the form of the desired cation, preferably sodium or potassium, and eluting with water. Furthermore, esters can be made in accordance with known procedures.

Any assimilable carbon source may be employed for fermentation, including a polyol or a carbohydrate, but more especially a chemically defined carbon source (that is, a carbon compound of chemically known structure). The carbon source typically has a molecular weight of below 500, and suitably comprises up to 12 carbon atoms in the molecule, such as in a mono- or di-saccharide, or a linear polyol. Examples of such compounds include maltose and glycerol. In an alternative, a suitable assimilable carbon source is a dextrin, starch or dextrinized starch. More generally, the carbon source is usually water soluble or water miscible. Solid carbon sources are preferably used in aqueous solution, while liquid sources may be used directly.

The use of S. clavuligerus NRRL 3585 is currently preferred. For such a micro-organism, the preferred carbon sources include maltose, glycerol, dextrins, and optionally dextrinized starches. Glucose, fructose, sucrose and lactose are not so readily assimilable by S. clavuligerus, and are therefore less preferred for such micro-organisms.

The fermentation medium can contain other suitable culture components, including an organic nitrogen source. Such nitrogen sources can comprise oleaginous seed, optionally defatted meals, protein hydrolysates or extracts. Mineral salts (particularly chlorides, sulphates, carbonates or phosphates) and defoaming agents of mineral or organic origin can be added where appropriate, together with other known medium components.

The optimum fermentation temperature is ordinarily between 24 and 30 °C, particularly from 26 to 28 °C, but fermentation can take place above or below these limits. The fermentation is typically effected on a batch system taking from 1 to many days, more usually from 24 to 240 hours, and especially 100 to 200

hours.

The most suitable fermentation vessel is a conventional aerobic fermentation vessel provided with means for agitation and aeration. The volume of such vessels can vary for instance from 1 liter and 225 $m^3$. The working volume is preferably from 25 to 75% of the total capacity.

In a preferred embodiment, in addition to lithium salt formation, the process of the present invention includes extraction of the clavulanic acid using ion-exchange resins which permit a higher exchange resin adsorption capacity. Examples of suitable resins are described, for instance, in UK patent 1,543,563. The resin is preferably a weak basic ion exchange resin of the IRA-68 type and is preferably in the form of the acetate.

The fermentation broth suitably has an initial activity of more than 800 µg/ml of clavulanic acid, in order to obtain a good crystallization of the lithium clavulanate, after adsorption of the broth in the ion exchange resin, elution thereof with a lithium salt and subsequent crystallization for example from isopropanol.

Before adsorption of the broth in the resin, it is best to effect a clarification.

In accordance with a further preferred mode of operating the present invention, the fermentation broth is first treated with an aggregating agent to aggregate the mycelium.

Aggregation of the mycelium permits a better filtration. A suitable aggregating agent is the filter coadjuvant Praestol (trademark of Bayer AG, Germany). The coadjuvant is preferably added to the broth at a concentration of from 0.1 to 0.5%, and the mix stirred for about 15 minutes before filtration. The filtration itself is suitably effected by passage through a bed of inorganic filter material such as Dicalite (typically about 6% w:v) in a Buchner funnel (Dicalite is a trademark of Dicalite Ltd). If the first volumes of filtration are not well clarified, Refiltration through the same Dicalite prelayer can be employed. The broth can also be clarified, after filtration, by centrifugation (typically 30 minutes at 10,000 rpm).

Another improvement in the operation of the method of this invention is an increase in the adsorption capacity of the ion-exchange resin achieved by deproteinization of the filtered broth.

The deproteinization is preferably effected by acidification, usually to around pH 4, or by treatment with a precipitating solvent such as acetone.

In an embodiment of the invention, the pH of the filtered broth is adjusted to about 4.0 with mineral acid, such as hydrochloric or sulphuric acid. Once the pH has been adjusted, the supernatant is adsorbed in the resin. The broth is preferably first allowed to stand, and any turbidity eliminated by centrifugation. To prevent product losses due to the pH of around 4, the mixture can be brought back to about pH 6 before adsorption on the resin. The adsorption capacity of the resin in the form of acetate at a pH of 6 is increased in this manner. Preferably the ratio of filtered broth:resin (v/v) is not more than 10:1

The filtered broth can also be deproteinized by treatment with a precipitating solvent, such as acetone. In an embodiment of the present invention, one volume of broth is mixed with about one volume of acetone. For preference, the broth is stirred for 5 to 10 minutes with acetone, allowed to stand for 20 minutes, and the precipitate separated by centrifugation or filtration. The broth thus clarified is suitable for adsorption in the resin. The acetone can be recoverable by distillation under reduced pressure before adsorption of the broth. Whether the acetone has been removed or not, the filtered broth can be adsorbed in the resin at a ratio of from 10:1 to 12:1 (initial volume of filtered broth:volume of resin) without considerable losses.

Crystallization of lithium clavulanate from the eluates of ion exchange resins has previously led to formation of gums and in some cases a low purity. To minimize this problem, it is preferred to wash or pre-elute the resin with 0.5% sodium chloride and then with water, before elution with the lithium salt, such as 5% lithium chloride.

Crystallization of the lithium clavulanate can be further improved by treatment of the eluate with acetone. The eluate of the resin is stirred for example with two volumes of acetone and allowed to stand for 15 minutes. The precipitate is separated by centrifugation to allow crystallization of the lithium clavulanate from isopropanol, after the acetone has been eliminated.

The present invention is illustrated in non-limiting manner by the following examples.

## EXAMPLE 1

The following medium was prepared:

fishmeal 2.0 g
glycerol 1.5 g
soluble starch 1.5 g
calcium carbonate 0.2 g

distilled water to 100 ml

The pH was adjusted to 7, and 40 ml aliquots were placed in 250 ml Erlenmeyer flasks and sterilized.

The medium was then seeded with a suspension of spores of S. clavuligerus NRRL 3585 and incubated for 2 days at 28 °C under agitation. The culture was employed to seed, to 2%, 500ml of the same medium in a 2-liter Erlenmeyer flask. Culturing was also carried out on a rotary shaker at 28 °C for 2 days.

500 ml of the culture thus prepared were used to seed a 340-liter stainless steel vessel fitted for agitation, aeration and temperature control. The vessel contained 100 liters of a sterilized medium of the following composition:

| | % |
|---|---|
| soybean meal | 3 |
| corn dextrin | 3 |
| soybean oil | 0.5 |
| $KH_2PO_4$ | 0.1 |
| defoamer (ucon) | 0.005 |
| tap water to final volume | |

The seeded medium was cultured at 28 °C, receiving air with stirring for 45 hours. Thereafter, a further 340-liter vessel was seeded, to 7%, with 150 liters of the same medium. Incubation took place at 28 °C for 30 hours, under stirring and aeration.

Upon termination, this culture was used to seed, to 7%, a clavulanic acid production medium contained in a tank. The tank had a total capacity of 800 liters, and 425 liters of the following medium were added:

soybean meal 1.25 %
peanut meal 1.25 %
distiller's dried grain 0.5 %
$KH_2PO_4$ 0.1%
defoamer (ucon) 0.005 %
tap water to final volume

Sterilization took place at 121 °C for 20 minutes, after adjusting the pH to 6.7. Glycerol was added intermittently every hour as follows:

| time (h) | rate (ml/hr) | total glycerol |
|---|---|---|
| 0 to 12 | 180 | 0.5% |
| 13 to 96 | 190 | 3.7% |
| 97 to 160 | 100 | 1.5% |

Fermentation was conducted at 26 °C under stirring and aeration. After 160hrs the level of clavulanic acid was high, at 1403 µg/ml.

## EXAMPLE 2

7.5 Liters of n-butanol were added to 7.5 liters of filtered broth from Example 1, and the pH was adjusted to 2 with cold 4N sulphuric acid. The mixture was centrifuged to 16,000 g, thereby separating the two phases, aqueous and butanolic. The butanol phase was concentrated under reduced presure at 35 °C to a volume of 200 ml.

In this way, a solution of clavulanic acid in 200 ml of n-butanol was obtained with a concentration of 26.7 mg/ml. 5 ml of water were added, giving a moisture level of 2.52%. To this solution, under stirring, were added 100 ml of 10% solution of lithium 2-ethylhexanoate in n-butanol. The total volume was concentrated to 150 ml, it was stirred for 2 hours at 5 °C and filtered through a filtering plate. The crystalline form was washed with 100 ml of isopropanol, 100 ml of acetone and 100 ml of ether, followed by drying at 40 °C under vacuum, to give 7.57 g of the product, purity 54%, moisture 3.2% and 23.5% ash (as lithium sulphate).

EXAMPLE 3

10 g of lithium clavulanate obtained as in Example 2 were.dissolved in 50 ml of water, adding thereto 75 ml of a solution of 50 w/v of lithium chloride slowly under stirring. Crystallization started, and the mix was stirred for half an hour at 5° C and filtered through a glass flask, washed with acetone and dried under vacuum at 40° C, obtaining 5.27 g of product, yield 84% amd purity 80%.

Physical characteristics of the recrystallized lithium clavulanate

R ($cm^{-1}$, Nujol (trade mark)): 3420, 3010, 1765, 1680, 1620, 1400, 1340, 1325, 1300, 1220, 1200, 1130, 1100, 1060, 1050, 1020, 990, 970, 950, 900, 880, 850, 730, 708.

$^{20}[\alpha]_D$ = 451 (c = 145 µg/ml in water)

UV $\lambda_{max}$ = 259 (c = 10 µg/ml in O.lN NaOH) (extinction molar coefficient, $\epsilon$ = 19,451) lithium content (by absorption) = 3.3% ash = 26.5%, as lithium sulphate.

## Claims

1. A process for the preparation of clavulanic acid wherein the clavulanic acid is purified from an optionally partially-purified fermentation broth using the steps of

(i) mixing the broth with dissolved lithium 2-ethylhexanoate;

(ii) isolating lithium clavulanate; and

(iii) optionally converting the lithium salt to another salt or to an ester.

2. A process according to claim 1 wherein the acid and lithium 2-ethylhexanoate are first dissolved in water-immiscible solvents, preferably the same solvent.

3. A process according to claim 2 wherein the water-immiscible solvent is n-butyl alcohol, ethyl acetate, methyl acetate, amyl acetate and/or methyl isobutyl ketone.

4. A process according to any preceding claim, wherein the clavulanic acid is purified using an ion-exchange resin.

5. A process according to any preceding claim, wherein the fermentation broth is treated with a filtration coadjuvant to aggregate the mycelium.

6. A process according to any preceding claim, wherein the fermentation broth is filtered and deproteinized.

7. A process according to any preceding claim, wherein the clavulanic acid is purified using an ion-exchange resin.

8. A process according to any preceding claim, wherein the fermentation broth is treated with a filtration coadjuvant to aggregate the mycelium.

9. A process according to any preceding claim, wherein the fermentation broth is filtered and deproteinized.